# EUROPEAN PATENT APPLICATION

(11) **EP 4 825 411 A1**
(43) Date of publication of application: **21.10.2026**
(21) Application number: 25170258.5
(22) Date of filing: 14.04.2025
(51) Int. Cl.: A61M 5/168

(54) **FLOW REGULATOR DEVICE FOR A MEDICAL PUMP**

(71) Applicant: B. Braun Melsungen AG, 34212 Melsungen (DE)
(72) Inventor: Weiß, André, 34225 Baunatal (DE); Weith, Florian, 14513 Teltow (DE); Khokhani, Paree, 61348 Bad Homburg vor der Höhe (DE)
(74) Representative: Patentanwälte Ruff, Wilhelm, Beier, Dauster & Partner mbB

(57) **Abstract**

A flow regulator device for a medical pump, the flow regulator device comprising: a housing having an inlet configured for fluid connection to an outlet of the medical pump and an outlet configured for fluid connection to an infusion line, a flow regulator body disposed in the housing between the inlet and the outlet, wherein the flow regulator body comprises an elastically compressible porous material allowing a fluid flow between the inlet and the outlet and configured for elastic compression by a fluid pressure gradient between the inlet and the outlet, wherein a fluid flow resistance of the flow regulator body increases with increasing elastic compression of the elastically compressible porous material and thus with increasing fluid pressure gradient, and wherein the fluid flow resistance of the flow regulator body decreases with decreasing elastic compression of the elastically compressible porous material and thus with decreasing fluid pressure gradient.

## Description

### TECHNICAL FIELD AND PRIOR ART

This invention relates to flow regulation in medical devices, in particular to a flow regulator device for a medical pump, e.g. an elastomeric infusion pump.

The continuous delivery of low flow rates over extended time periods presents a challenge for medical pumps, in particular for elastomeric infusion pumps. Currently, solutions such as polymer-based microbore tubes with small inner diameters or glass capillaries are integrated into such medical pumps to achieve low flow rates. However, these solutions are difficult to manufacture and often result in inconsistent flow rates, leading to blockages within the medical pump. Other solutions, which are claimed to be robust and efficient, include microchannel or micro-capillary chip-based components. However, such solutions are expensive and tend to be bulky, reducing customer acceptance. Additionally, devices like roller clamps, mechanical actuators, and flow selector devices that operate on the principle of pinching tubes also contribute to flow regulation but face challenges in maintaining low flow rates.

US 2022/0126018 A1, for example, discloses an elastomeric infusion pump having a flow regulator device with a fluid channel, wherein an effective length of the channel is variable for increasing and decreasing a fluid flow resistance of the channel.

### SUMMARY OF THE INVENTION

It is an object of the present invention to provide a flow regulator device having improved properties. In particular, it is an object to provide a flow regulator device having a simple, small and lightweight design while at the same time allowing for a robust regulation of fluid flow.

This object is solved by providing a flow regulator device comprising the features of claim 1.

According to the invention, the flow regulator is for a medical pump and comprises a housing having an inlet configured for fluid connection to an outlet of the medical pump, an outlet configured for fluid connection to an infusion line, and a flow regulator body disposed in the housing between the inlet and the outlet, wherein the flow regulator body comprises an elastically compressible porous material allowing a fluid flow between the inlet and the outlet and configured for elastic compression by a fluid pressure gradient between the inlet and the outlet, wherein a fluid flow resistance of the flow regulator body increases with increasing elastic compression of the elastically compressible porous material and thus with increasing fluid pressure gradient, and wherein the fluid flow resistance of the flow regulator body decreases with decreasing elastic compression of the elastically compressible porous material and thus with decreasing fluid pressure gradient. The invention allows for self-adjusting regulation of the fluid flow while providing a robust and simple design of the flow regulator device. The fluid flow resistance is variable and depends on the elastic compression of the elastically compressible porous material (in the following: ECP material). The elastic compression depends on the fluid pressure gradient between the inlet and the outlet. The higher the fluid pressure at the inlet (in comparison to the fluid pressure at the outlet), the higher the elastic compression and thus the fluid flow resistance. The lower the fluid pressure at the inlet (in comparison to the fluid pressure at the outlet), the lower the elastic compression and thus the fluid flow resistance. Consequently, the fluid flow resistance of the flow regulator body varies with varying fluid pressure gradient which allows for self-adjusting regulation of the fluid flow. In different embodiments, the housing has different forms and/or shapes. In principle, the housing can have any form and/or shape suitable for the present purpose. The housing accommodates the flow regulator body. The flow regulator body is disposed in the housing and between the inlet and the outlet. During operation of the flow regulator device, the fluid flow enters the housing through the inlet, flows through the flow regulator body, in particular the ECP material, and exits the housing through the outlet. In different embodiments, the flow regulator body comprises and/or is made of different ECP materials. The ECP material is elastic, compressible and porous. In one embodiment at least a portion of the flow regulator body comprises or is made of the ECP material. In one embodiment. In one embodiment, the flow regulator body comprises a mixture, laminate and/or arrangement of different ECP materials. In one embodiment, the flow regulator body comprises and/or is made of a single ECP material.

In one embodiment, the ECP material comprises a foamed material. Alternatively, the ECP material is a foamed material. The foamed material is elastic, compressible and porous. Preferably, the foamed material is a foamed plastics material, the plastics material, e.g., comprising polyolefine, styrene polymer and/or polyurethane.

In one embodiment, the ECP material comprises a sintered material. Alternatively, the ECP material is a sintered material. The sintered material is elastic, compressible and porous. Preferably, the sintered material is a sintered metal and/or plastics material.

In one embodiment, the ECP material comprises a natural material. Alternatively, the ECP material is a natural material. Preferably, the natural material is a natural sponge.

In one embodiment, the ECP material has a porosity of 10% to 95%, preferably of 50% to 75%. Porosity or void fraction is a measure of the void spaces in a material and is a fraction of the volume of voids over the total volume, between 0 and 1, or as a percentage between 0% and 100%.

In one embodiment, the ECP material comprises pores having pore sizes from 1 nm to 100 µm, preferably from 1 nm to 100 nm, more preferably from 100 nm to 1 µm, more preferably from 1 µm to 10 µm, more preferably from 10 µm to 100 µm. The pore sizes specified relate to an uncompressed configuration of the ECP material, while compression may lead to decreasing pore sizes and/or lowered porosity. The specified pore size value ranges can be reliably and robustly produced in a simple manner using conventional foaming and/or sintering processes, in particular when metal and/or plastic is used.

In one embodiment, the housing comprises a cone shaped portion comprising the outlet and forming a conical seating surface for the flow regulator body, the conical seating surface providing a radial compression component upon an axial compression of the flow regulator body against the conical seating surface. The conical seating surface contributes to the elastic compression by providing said radial compression component upon axial compression of the flow regulator body. The fluid pressure gradient between the inlet and the outlet causes a compression in axial direction of the housing and/or the flow regulator body. Compressing the flow regulator body axially causes increased contact pressure between the flow regulator body and the conical seating surface, the increased contact pressure having a radial component that causes said radial compression component. The conical seating surface is adjacent to the outlet. Changing the design of the cone shaped portion and/or the conical seating surface, in particular its cone angle, allows adjusting the achievable flow rates. Faster flow rates can be achieved by designing a larger cone angle. Slower flow rates can be achieved by designing a smaller cone angle, i.e. by making the cone shaped portion and thus the conical seating surface more pointed (towards the outlet).

In one embodiment, the flow regulator device further comprises a biasing device configured for biasing the flow regulator body into different pre-compressed configurations. The biasing device allows pre-compressing the flow regulator body for predefining different fluid flow resistances. In one embodiment, the biasing device is connected to the housing and configured for manual adjustment between different biasing positions relative to the housing and/or the flow regulator body, thereby causing different pre-compressed configurations of the flow regulator body.

In one embodiment, the biasing device comprises a screw member reaching into the housing and configured for, preferably axial, pre-compression of the flow regulator body. Preferably, the screw member is manually screwable. Tightening the screw member causes an increasing pre-compression. Loosening the screw member causes a decreasing pre-compression.

In one embodiment, the flow regulator device further comprises a first check valve member disposed upstream of the flow regulator body and/or a second check valve member downstream of the flow regulator body. The first check valve member and/or the second check valve member avoid reflux, i.e. fluid flow entering the housing through the outlet and/or exiting the housing through the inlet. Moreover, the first check valve member and/or the second check valve member are configured to stabilize the position of the flow regulator body in the housing.

In one embodiment, the first check valve member and/or the second check valve member comprises an elastic membrane valve. Depending on the fluid pressure gradient, the elastic membrane valve is open or closed, i.e. enabling or inhibiting fluid flow. Upon reaching or exceeding a certain fluid pressure gradient threshold, the elastic membrane valve opens. The elastic membrane valve closes, if the fluid pressure gradient falls below said threshold. In a preferred embodiment, the elastic membrane valve has at least one slit forming an/the inlet into the housing or an/the outlet out of the housing, respectively.

The invention also relates to a medical pump for pumping medical fluids and/or body fluids, the medical pump comprising an outlet and a flow regulator device according to the present disclosure, wherein the outlet of the medical pump is fluidly connected to the inlet of the flow regulator device. In a preferred embodiment, the medical pump is an elastomeric infusion pump.

### BRIEF DESCRIPTION OF THE DRAWINGS

In the following, exemplary embodiments of the invention are described in detail with reference to the drawings. Throughout the drawings, same elements are denoted with same reference numerals. The drawings schematically show:
- fig. 1: an embodiment of a medical pump according to the invention, the medical pump comprising an embodiment of a flow regulator device according to the invention,
- fig. 2: the flow regulator device having a housing and a flow regulator body together with an enlarged detailed view of the flow regulator body,
- fig. 3, 4: the flow regulator body in an elastically compressed first configuration (fig. 3) and an elastically compressed second configuration (fig. 4),
- fig. 5: a further embodiment of a flow regulator device according to the invention, the hosing of the flow regulator device further comprising a cone shaped portion,
- fig. 6: a further embodiment of a flow regulator device according to the invention, the flow regulator device further comprising a biasing device, and
- fig. 7: a further embodiment of a flow regulator device according to the invention, the flow regulator device further comprising a first check valve member and a second check valve member.

### DETAILED DESCRIPTION OF EXEMPLARY EMBODIMENTS OF THE INVENTION

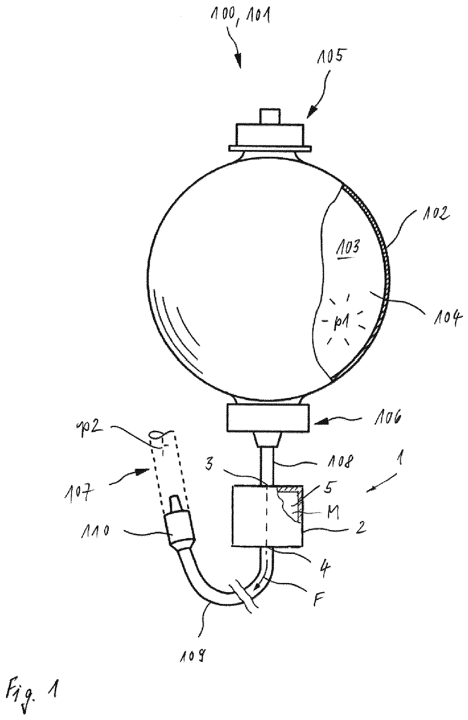

According to fig. 1, a medical pump 100 is provided for pumping medical fluids during outpatient and/or inpatient infusion therapy.

In the embodiment shown, the medical pump 100 is an elastomeric infusion pump 101.

The elastomeric infusion pump 101 comprises an elastomeric membrane 102 which forms a pump volume 103 containing a medical fluid 104. In the configuration shown in fig. 1, the elastomeric membrane 102 is elastically expanded into a balloon-like shape pressurizing the pump volume 103 and thus the medical fluid 104 with a pump pressure p1.

The elastomeric infusion pump 101 further comprises a resealable filling cap 105 for filling the medical fluid 104 into the elastomeric membrane 102 and the pump volume 103, respectively.

The elastomeric infusion pump 101 further comprises an outlet nozzle 106 connected to a connection line 108, 109 having a first connection line portion 108 and a second connection line portion 109. A proximal end of the first connection line portion 108 is connected to the outlet nozzle 106. A distal end of the second connection line portion 109 is connectable or connected to an infusion line 107 by means of a fluid connector 110.

During infusion therapy, the pump pressure p1 drives the medical fluid 104 from the pump volume 103 through the outlet nozzle 106 into the connection line 108, 109 and from there into the infusion line 107.

Fluid flow regulation is mandatory to ensure precise dosage of the medical fluid 104, i.e. to avoid over- and/or underdosing. For that purpose, the elastomeric infusion pump 101 further comprises a flow regulator device 1.

In the embodiment shown, the flow regulator device 1 is a component of the connection line 108, 109. In other embodiments, the flow regulator device 1 is integrated into the outlet nozzle 106.

The flow regulator device 1 comprises a housing 2 and a flow regulator body 5.

The housing comprises an inlet 3 and an outlet 4. In the embodiment shown, the inlet 3 is fluidly connected to the pump volume 103 via the outlet nozzle 106 and the first connection line portion 108. The outlet 4 is fluidly connected to the infusion line 107 via the second connection line portion 109 and the fluid connector 110.

The fluid connection of the inlet 3 and the outlet 4 can be fixed or releasable. For releasable fluid connection, the inlet 3 and/or the outlet 4 can comprise a fluid connector.

The flow regulator body 5 is positioned within the housing 2, between the inlet 3 and the outlet 4. During infusion therapy, the medical fluid 104 enters the housing 2 through the inlet 3, flows through the flow regulator body 5 and exits the housing (and the flow regulator body 5) through the outlet 4.

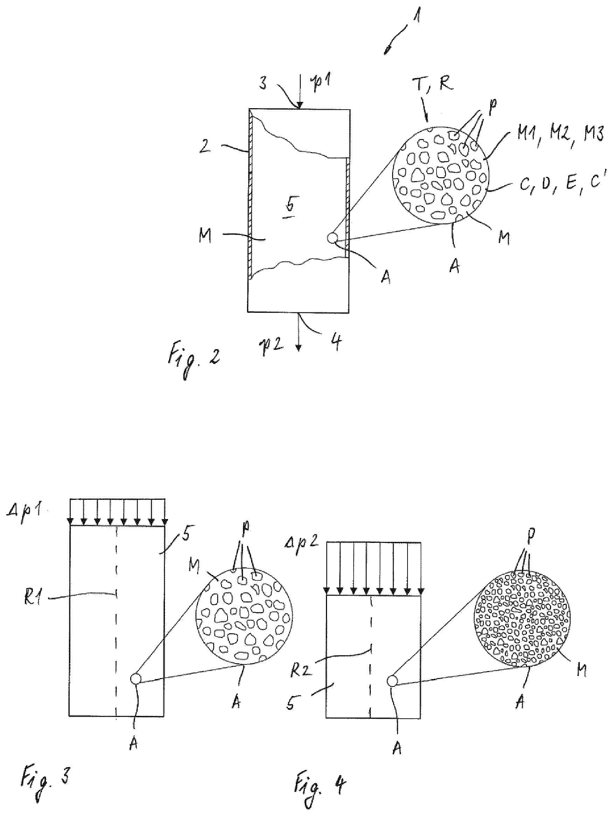

Referring to fig. 2, the flow regulator body 5 comprises an elastically compressible porous material M that allows said fluid flow F between the inlet 3 and the outlet 4. For sake of brevity, the elastically compressible porous material M is also referred to as ECP material M.

The ECP material M is configured for elastic compression by a fluid pressure gradient between the inlet 3 and the outlet 4. At the inlet 3, the medical fluid 104 has a first pressure or inlet pressure that roughly equals, in the present embodiment, the pump pressure p1. At the outlet 4, the medical fluid 104 has a second pressure or outlet pressure p2 that, in the present embodiment, roughly equals the pressure inside the infusion line 107. The fluid pressure gradient between the inlet 3 and the outlet 4 can also be referred to as pressure drop, expressed as Δp=p1-p2.

Because of the elastically compressible porous properties of the ECP material M, the flow regulator body 5 has a fluid flow resistance R that varies as a function of the elastic compression and thus the fluid pressure gradient Δp. Hence, the fluid flow resistance R of the flow regulator body 5 increases with increasing elastic compression of the ECP material M and thus with increasing fluid pressure gradient Δp. A decreasing fluid pressure gradient Δp causes decreasing elastic compression of the ECP material M and thus a decreasing fluid flow resistance R.

Consequently, the flow regulator device 1 allows for a self-adjusting flow regulation enabling continuous delivery of low flow rates for longer periods of time.

The ECP material M has foam- and/or spongelike properties. The ECP material has open pores P and can comprise or be made of different material, e.g. foamed material M1, sintered material M2 and/or natural material M3.

In one embodiment, the ECP material M comprises or is said foamed material M1, preferably a foamed plastics material C. For example, the foamed plastics material C comprises or is polyolefine, styrene polymer and/or polyurethane.

In one embodiment, the ECP material M comprises or is said sintered material M2, preferably a sintered metal D. Alternatively the sintered material M2 can be a sintered plastics material C'.

In one embodiment, the ECP material M is or comprises said natural material M3. Preferably, the natural material M3 is a natural sponge E.

The ECP material M has open pores and, in its uncompressed state, a porosity T of 10% to 95%. In the embodiment shown, the porosity T is 50% to 75%.

In the configuration shown in fig. 2, the ECP material M and thus the flow regulator body 5 is uncompressed, i.e. fully relaxed. In the embodiment shown, the uncompressed ECP material has pore sizes from 100 nm to 1 µm.

In embodiments not shown in the figures, the pore sizes range from 1 nm to 100 µm, 1 nm to 100 nm, 1 µm to 100 µm or 10 µm to 100 µm.

In a preferred exemplary embodiment, the ECP material M is a polymer material, comprises a porosity T of 50% and has a pore size of its open pores P that ranges from 1 µm to 10 µm.

Figs. 3 and 4 show exemplary elastically compressed configurations of the flow regulator body 5, wherein the flow regulator body 5 is compressed under the influence of a first fluid pressure gradient Δp1 (fig. 3) and a higher second fluid pressure gradient Δp2. The first fluid pressure gradient Δp1 compresses the ECP material M into a first compressed state, in which the ECP material M has a first fluid flow resistance R1 acting on the fluid flow F through the flow regulator body 5. The higher second fluid pressure gradient Δp2 compresses the flow regulator body 5 in a second compression state, in which the ECP material M has a higher second fluid flow resistance R2. The open pores P of the ECP material M change their size and/or form due to compression, which is schematically shown in the enlarged detail area A included in fig. 3, 4. In the second compressed state, the open pores P are smaller or further squeezed together as in the first compressed state.

In the embodiment according to fig. 2, 3, 4, the housing 2 has a cylindrical shape longitudinally extending in axial direction between the inlet 3 and the outlet 4. The flow regulator body 5 has a complementary cylindrical shape filling the housing 2 between the inlet 3 and the outlet 4. Under compression, the flow regulator body 5 changes its length in axial direction of the housing 2. The flow regulator body 5 is radially contained in the housing 2 and as a consequence of the complementary cylindrical shapes of both the housing 2 and the flow regulator body 5, there is no radial compression component.

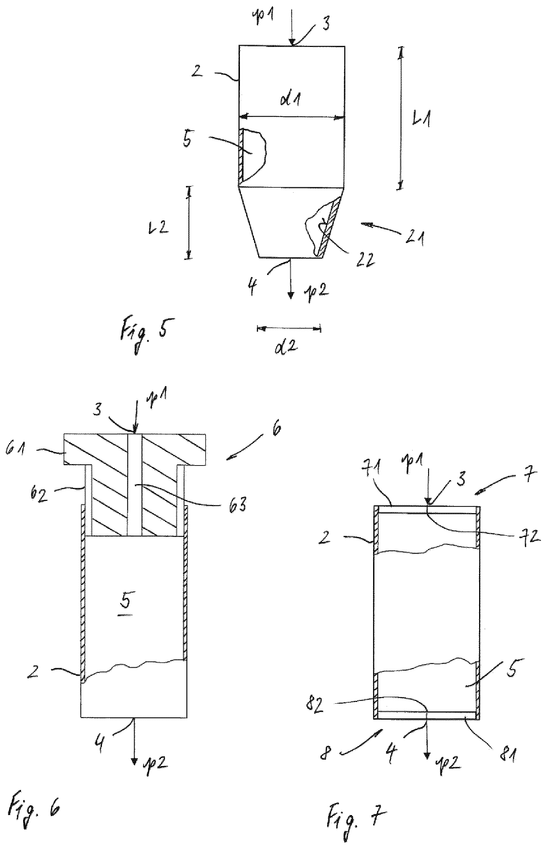

In the embodiment shown in fig. 5, the housing 2 comprises a cone shaped portion 21 forming a conical seating surface 22 for the flow regulator body 5. The conical seating surface 22 provides a radial compression component upon axial compression of the flow regulator body 5 against the conical seating surface 22. Hence, the fluid pressure gradient Δp presses the flow regulator body 5 in axial direction towards the outlet 4 of the housing 2 and onto the conical seating surface 22. As a consequence of the conical shape of the conical seating surface 22, the flow regulator body 5 is also compressed in radial direction, thereby causing a stronger overall compression and therefore more pronounced change of fluid flow resistance. The shape and size of the cone shaped portion 21 affects the flow regulating properties. Design changes to the shape and form of the cone shaped portion 21 enable simple adaptation of these properties.

In the embodiment according to fig. 5, the housing 2 has an overall axial length L1+L2, wherein L1 is a first length of a cylindrical portion of the housing, and L2 is a second length of the cone shaped portion 21. The cylindrical portion has a first diameter d1. The cone-shaped portion 21, at its distal, pointed end, has a second diameter d2. The larger the second diameter d2 relative to the first diameter d1, the faster the flow rate. Conversely, the smaller the second diameter d2 relative to the first diameter d1, or the more pointed the cone shaped portion 21, the slower the flow rate.

In the embodiment according to fig. 6, the flow regulator device 1 further comprises a biasing device 6 configured for biasing the flow regulator body 5 into different pre-compressed configurations.

In the embodiment shown, the biasing device 6 comprises a screw member 61. The screw member 61 comprises a threaded portion 62 and an inlet channel 63. A proximal end of the inlet channel 63 forms the inlet 3 of the housing 2. A distal end of the inlet channel 63 leads directly into the housing 2. The threaded portion 62 is engaged with a threaded portion (without reference sign) of the housing 2.

In the embodiment shown, the screw member 61 is axially screwed into the proximal end of the housing 2. Tightening the screw member 61 compresses the flow regulator body 5 along the axial direction. Loosening the screw member 61 relaxes the flow regulator body 5 in axial direction.

In the embodiment according to fig. 7, the flow regulator device further comprises a first check valve member 7 and a second check valve member 8. The first check valve member 7 is disposed at the inlet and hence upstream of the flow regulator body 5. The second check valve member 8 is disposed at the outlet 4 and hence downstream of the flow regulator body 5.

The first check valve member 7 and the second check valve member 8 are configured to allow fluid flow in the regular direction, i.e., from the inlet 3 towards the outlet 4, and to inhibit reflux in the opposite direction.

In the embodiment shown, the first check valve member 7 is disposed at the proximal end of the housing 2 forming an upper or proximal seal. The second check valve member is disposed at the distal end of the housing 2 forming a distal or lower seal. In other words: The first check valve member 7 forms an upper end cap of the housing 2 and the second check valve member 8 forms a lower end cap of the housing 2. In the embodiment shown, the flow regulator body 5 is positioned between the first check valve member 7 and the second check valve member 8.

In the embodiment according to fig. 7, the first check valve member 7 and the second check valve member 8 each comprise an elastic membrane valve 71, 81, which are also referred to as first elastic membrane valve 71 and second elastic membrane valve 81.

The first elastic membrane valve 71 comprises a slit forming the inlet 3 of the housing 2. The second elastic membrane valve 81 comprises a slit 82 forming the outlet 4 of the housing 2. Said slits 72, 82 are configured to open upon reaching or exceeding a defined fluid pressure threshold. A pressure drop below said fluid pressure threshold causes the slits 72, 82 to close.

## Claims

1. A flow regulator device (1) for a medical pump (100, 101), the flow regulator device (1) comprising
a housing (2) having an inlet (3) configured for fluid connection to an outlet (106) of the medical pump (100, 101) and an outlet (4) configured for fluid connection to an infusion line (107),
a flow regulator body (5) disposed in the housing (2) between the inlet (3) and the outlet (4),
wherein the flow regulator body (5) comprises an elastically compressible porous material (M) allowing a fluid flow (F) between the inlet (3) and the outlet (4) and configured for elastic compression by a fluid pressure gradient (Δp) between the inlet (3) and the outlet (4),
wherein a fluid flow resistance (R) of the flow regulator body (5) increases with increasing elastic compression of the elastically compressible porous material (M) and thus with increasing fluid pressure gradient (Δp),
and wherein the fluid flow resistance (R) of the flow regulator body (5) decreases with decreasing elastic compression of the elastically compressible porous material and thus with decreasing fluid pressure gradient (Δp).

2. The flow regulator device (1) according to claim 1, wherein the elastically compressible porous material (M) comprises or is a foamed material (M1), preferably a foamed plastics material (C).

3. The flow regulator device (1) according to claim 1 or 2, wherein the elastically compressible porous material (M) comprises or is a sintered material (M2), preferably a sintered metal (D) and/or a sintered plastics material (C').

4. The flow regulator device (1) according to any of the preceding claims, wherein the elastically compressible porous material (M) comprises or is a natural material (M3), preferably a natural sponge (E).

5. The flow regulator device (1) according to any of the preceding claims, wherein the elastically compressible porous material (M) has a porosity (T) of 10% to 95%, preferably of 50% to 75%.

6. The flow regulator device (1) according to any of the preceding claims, wherein the elastically compressible porous material (M) comprises pores (P) having pore sizes from 1 nm to 100 µm, preferably from 1 nm to 100 nm, more preferably from 100 nm to 1 µm, more preferably from 1 µm to 10 µm, more preferably from 10 µm to 100 µm.

7. The flow regulator device (1) according to any of the preceding claims, wherein the housing (2) comprises a cone shaped portion (21) comprising the outlet (4) and forming a conical seating surface (22) for the flow regulator body (5), the conical seating surface (22) providing a radial compression component upon an axial compression of the flow regulator body (5) against the conical seating surface (22).

8. The flow regulator device (1) according to any of the preceding claims, further comprising a biasing device (6) configured for biasing the flow regulator body (5) into different pre-compressed configurations.

9. The flow regulator device (1) according to claim 8, wherein the biasing device (6) comprises a screw member (61) reaching into the housing (2) and configured for, preferably axial, pre-compression of the flow regulator body (5).

10. The flow regulator device (1) according to any of the preceding claims, further comprising a first check valve member (7) disposed upstream of the flow regulator body (5) and/or a second check valve member (8) disposed downstream of the flow regulator body (5).

11. The flow regulator device (1) according to claim 10, wherein the first check valve member (7) and/or the second check valve member (8) each comprise an elastic membrane valve (71, 81).

12. A medical pump (100, 101) for pumping medical fluids and/or body fluids, the medical pump (100, 101) comprising an outlet (106) and a flow regulator device (1) according to any of the preceding claims, wherein the outlet (106) is fluidly connected to the inlet (3) of the flow regulator device (1).
